Europäisches Patentamt

**European Patent Office**

**Office européen des brevets**

(11) Numéro de publication: **0 240 432 B1**

(12) **FASCICULE DE BREVET EUROPEEN**

(45) Date de publication de fascicule du brevet: **03.03.93** (51) Int. Cl.5: **G01N 33/14**

(21) Numéro de dépôt: **87400726.3**

(22) Date de dépôt: **02.04.87**

Le dossier contient des informations techniques présentées postérieurement au dépôt de la demande et ne figurant pas dans le présent fascicule.

(54) **Procédé et installation pour l'étude, en fonction du temps, des caractéristiques d'un chapeau de mousse de bière.**

(30) Priorité: **04.04.86 FR 8604852**

(43) Date de publication de la demande:
**07.10.87 Bulletin 87/41**

(45) Mention de la délivrance du brevet:
**03.03.93 Bulletin 93/09**

(84) Etats contractants désignés:
**AT BE CH DE ES GB IT LI LU NL SE**

(56) Documents cités:
**DE-A- 2 734 888**
**DE-A- 3 022 848**
**FR-A- 2 336 674**
**GB-A- 2 161 601**

(73) Titulaire: **CENTRE DE RECHERCHES ET DE DEVELOPPEMENT DE TECHNIOUES ET PRO-DUITS ALIMENTAIRES TEPRAL SA**
**20, rue Jacob**
**F-67200 Strasbourg(FR)**

(72) Inventeur: **Carnielo, Michel**
**2, Allée des Bouleaux Résidence Bois le Duc**
**F-54500 Vandoeuvre(FR)**
Inventeur: **Oortwijn, Jan**
**23, rue de Wissenbourg**
**F-67300 Schiltingheim(FR)**

(74) Mandataire: **Orès, Bernard et al**
**Cabinet ORES 6, Avenue de Messine**
**F-75008 Paris (FR)**

Rank Xerox (UK) Business Services
(3.10/3.5x/3.0.1)

## Description

L'invention a pour objet un procédé et une installation pour l'étude, en fonction du temps, des caractéristiques d'un chapeau de mousse de bière.

Elle vise, en particulier, un procédé et une installation permettant de mesurer la modification des caractéristiques d'un chapeau de mousse de bière qui apparaît dans un récipient transparent comme un verre ou une chope lorsque la bière y est versée à partir d'une bouteille, d'une canette, ou par soutirage à partir d'un fût.

On sait qu'un tel chapeau de mousse disparaît plus ou moins rapidement après avoir été formé, de manière d'ailleurs non uniforme dans le verre, et qu'à cette plus ou moins grande vitesse de disparition sont attachées des différences d'appréciation qualitative par le consommateur, généralement favorable dans le cas d'une relative persistance du chapeau de mousse et défavorable dans le cas contraire.

Le phénomène est suffisamment important pour que les brasseries surveillent les caractéristiques de formation et d'évolution des chapeaux de mousse au cours de la fabrication de la bière, ou au cours de dégustations, l'examen étant jusqu'à présent généralement limité à l'observation du temps de disparition d'un chapeau de mousse formé après que la bière ait été versée dans un verre à partir d'une bouteille ou par soutirage d'un fût.

Il en est ainsi, par exemple, dans FR-A-2 336 674 qui décrit un procédé et un appareil de mesure du temps de résorption d'un chapeau de mousse de bière à l'aide d'un dispositif photoélectrique dont la source lumineuse est placée sous le tube dans lequel est versée la bière, le résultat de la mesure étant exprimé par le temps de disparition de la couronne de mousse lorsqu'apparaît une zone transparente au rayonnement dans le tube. Le mode opératoire, notamment pour transvaser la bière est relativement long de sorte que le procédé est d'emploi difficile. En outre, il ne donne qu'une indication par "tout ou rien".

Il en est de même dans le procédé et l'appareil décrits dans GB-A-2 161 601 qui prévoit également un dispositif photoélectrique dirigé suivant l'axe du verre dans lequel est versée la bière.

Dans le procédé selon DE-A-2 734 888, les essais sont conduits dans une éprouvette de grande hauteur garnie d'un côté d'une pluralité de sources lumineuses et de l'autre d'un même nombre de photocapteurs, les sources et les photocapteurs étant reliés à un ensemble de traitement électronique. La multiplicité de sources lumineuses et de capteurs rend ici aussi l'exploitation difficile et cela d'autant plus que chaque essai doit être conduit à l'aide d'une éprouvette spéciale et ainsi dans des conditions différentes de celles d'une dégustation par un consommateur usuel.

Dans DE-A-3 022 848 un système de mesure photo-électrique dont la source et le récepteur sont placés dans un plan perpendiculaire à l'axe d'une éprouvette est prévu sur un équipage mobile se déplaçant le long de l'éprouvette dans laquelle est versée la bière. En raison des nombreux déplacements dudit équipage qu'exige la mesure, le fonctionnement de l'appareil n'est pas non plus sans aléas ; en outre, si le niveau de l'interface de la mousse et de la bière peut être déterminé avec relativement de précision, il n'en est pas toujours de même du niveau haut du chapeau de mousse qui est souvent masqué par les restes ou dentelle de mousse sur la paroi du verre.

Le besoin existe, par conséquent, de fournir des moyens appropriés pour établir des conditions reproductibles d'expérience permettant l'obtention de résultats chiffrés comparables entre eux et/ou l'exploitation d'un procédé suffisamment rigoureux pour former la base d'une exploitation industrielle.

C'est, par conséquent, un but de l'invention de fournir un procédé et une installation pour l'étude, en fonction du temps, des caractéristiques d'un chapeau de mousse de bière qui permettent de s'affranchir des inconvénients mentionnés ci-dessus.

C'est, à cet égard, un but de l'invention de fournir un procédé et une installation qui procurent les mêmes conditions expérimentales quels que soient les essais effectués et qui, ainsi, en assurant le caractère reproductible des paramètres mis en jeu permettent d'établir une analyse quantitative des phénomènes observés.

C'est, aussi, un but de l'invention de fournir un procédé et une installation à traitement automatique des résultats obtenus en ce qui concerne l'évolution, dans le temps, des caractéristiques du chapeau de mousse de bière.

Un procédé selon l'invention, pour l'étude en fonction du temps de la variation de hauteur d'un chapeau de mousse de bière apparaissant à la partie supérieure d'un récipient transparent comme un verre ou analogue après qu'y ait été versée la bière à partir d'un récipient comme une bouteille, une canette ou par soutirage d'un fût suivant lequel on détermine à l'aide de moyens optiques la hauteur de l'interface bière/mousse et celle du chapeau dans le récipient disposé verticalement, est caractérisé en ce que l'on projette sur le sommet du chapeau de mousse et selon un axe vertical un faisceau lumineux, en ce que l'on forme simultanément à des intervalles de temps prédéterminés et par des prises de vue effectuées transversalement au récipient des images du sommet dudit chapeau et du niveau de l'interface bière/mousse, et en ce que l'on traite lesdites images pour obtenir les variations des caractéristiques du chapeau de mousse, en particulier la vitesse de disparition dudit chapeau.

Dans une forme de réalisation préférée, le faisceau lumineux projeté sur le sommet du chapeau de mousse est issu d'une fente rectiligne étroite et l'on traite alors les images de ladite fente sur le sommet du chapeau avec celles du niveau de l'interface bière/mousse pour obtenir les variations des caractéristiques du chapeau de mousse.

Les images sont des images photographiques obtenues à l'aide d'un flash.

Dans une réalisation particulièrement avantageuse, les images sont des images optoélectroniques, notamment des images de télévision.

Un tel procédé, qui ne détruit pas la texture et la structure du chapeau de mousse observé, est d'une exploitation particulièrement intéressante et traduit mieux les phénomènes observés que les procédés connus décrits ci-dessus.

Une installation pour l'étude, en fonction du temps, des caractéristiques d'un chapeau de mousse de bière apparaissant à la partie supérieure d'un récipient transparent comme un verre ou analogue après qu'y ait été versée la bière à partir d'un récipient comme une bouteille, une canette, ou par soutirage d'un fût, avec des moyens optiques pour déterminer le niveau de l'interface bière/mousse et la hauteur du sommet du chapeau de mousse dans le récipient disposé verticalement, est caractérisée en ce que lesdits moyens optiques comprennent, d'une part une source d'éclairement à diaphragme qui projette sur le sommet du chapeau de mousse et suivant un axe vertical un faisceau lumineux et, d'autre part, des moyens de formation d'images propres à effectuer des prises de vue transversalement au récipient dudit niveau de l'interface bière/mousse à des instants pré-établis et des moyens de traitement desdites images associés auxdits moyens de formation d'images.

Dans une forme de réalisation préférée, l'installation comprend en outre un appareillage pour remplir le verre ou analogue de manière prédéterminée et reproductible, ledit appareillage comportant un équipage en forme générale de lutrin à deux versants, susceptible d'être animé d'un mouvement de pivotement autour d'un axe parallèle à l'intersection des deux versants sous l'action d'un moteur de commande à vitesse réglable.

L'équipage pivotant comprend avantageusement des moyens de positionnement relatif à la bouteille et du verre, ainsi que des moyens de réglage de la distance relative de l'un et de l'autre.

Les moyens optiques comprennent des organes propres à définir optiquement la partie supérieure du chapeau de mousse.

Dans une forme de réalisation préférée, lesdits organes comprennent un diaphragme linéaire à fente étroite pour la projection d'un faisceau lumineux de forte intensité sur la partie supérieure du chapeau de mousse.

Dans une réalisation, les moyens formateurs d'images comprennent un appareil photographique dont le déclenchement est synchrone de celui de moyens de flash, l'ensemble étant commandé suivant une séquence préprogrammée à l'aide d'un calculateur ou analogue.

Dans une variante préférée, les moyens formateurs d'images sont des moyens optoélectroniques, avantageusement du type télévision, et la variation des caractéristiques du chapeau de mousse en fonction du temps, en particulier la vitesse de diminution de la hauteur dudit chapeau, est déterminée par des moyens de calcul automatique auxquels sont associés, le cas échéant, des moyens traceurs de courbe.

L'invention sera bien comprise par la description qui suit, faite à titre d'exemple et en référence au dessin annexé dans lequel :

- la figure 1 est une vue schématique de dessus d'un appareillage d'une installation suivant l'invention, certaines parties ayant été ôtées dans un but de clarté ;
- la figure 2 est une vue schématique en coupe selon la ligne 2-2 de la figure 1, avec une bouteille et un verre ;
- la figure 3 est une vue analogue à celle de la figure 2, mais pour une autre condition ;
- la Figure 4 est une vue schématique d'une autre partie de l'installation selon l'invention,
- la Figure 5 est une vue schématique d'un verre rempli de bière et de mousse,
- la Figure 6 montre des courbes représentatives de résultats d'essais.

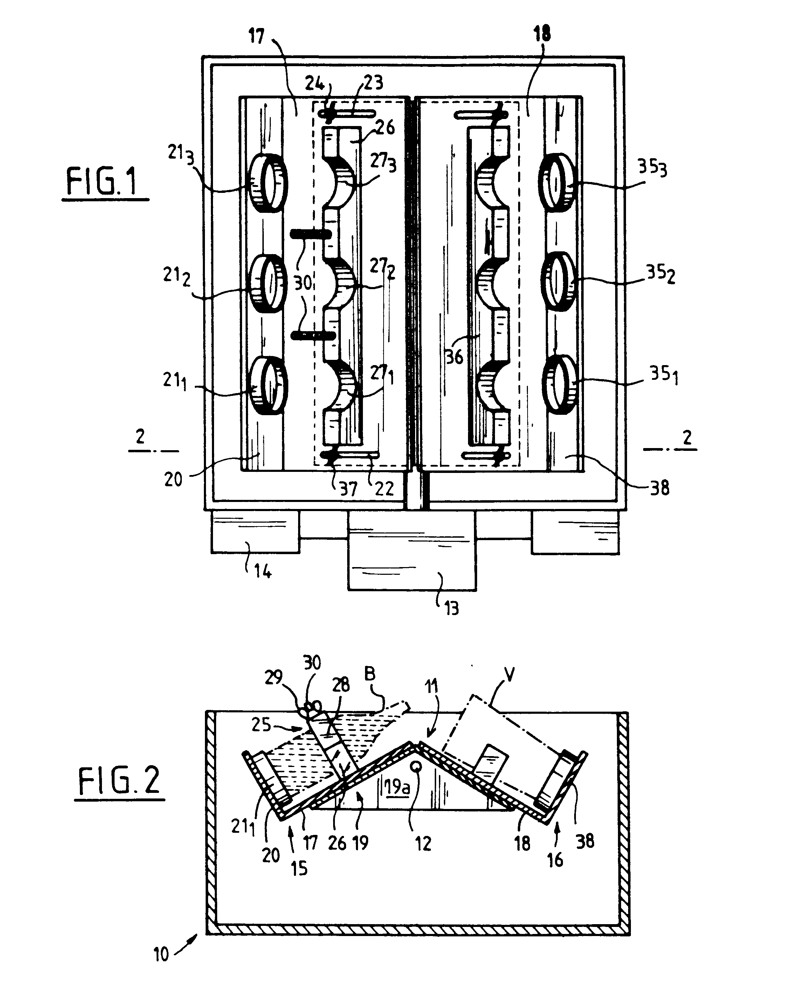

On se réfère d'abord aux Figures 1 à 3 qui illustrent un appareillage d'une installation suivant l'invention et qui est destiné à verser le contenu de canettes de bières dans des verres selon un processus prédéterminé à caractéristiques définies et reproductibles.

Un tel appareillage comprend, à l'intérieur d'un bac 10, un équipage 11 propre à être animé d'un mouvement de pivotement autour d'un axe 12 sous l'action d'un moteur électrique 13, lequel est commandé quant à son sens et sa vitesse de rotation à partir de moyens 14. L'équipage 11 est quelque peu en forme de lutrin à deux versants, c'est-à-dire comprend un premier et un second profilé en L, 15 et 16, respectivement, dont les ailes de grande dimension 17 et 18 sont de position réglable sur un support 19 à versants en ∧ réunis par des plaques triangulaires 19a traversées par l'axe 12, ainsi parallèle à l'intersection desdits versants.

Des boutonnières 22 et 23, ménagées dans les ailes 17 et 18 et qui sont traversées par des tiges filetées 24 solidaires du support 19 permettent le réglage en position des profilés 15 et 16 sur ledit support, des écrous à ailettes 37 coopérant avec les tiges filetées 24 étant prévus pour l'immobilisation des profilés 15 et 16 dans la position choisie.

Sur l'aile 20 de petites dimensions du profilé 15 sont fixées trois coupelles $21_1$, $21_2$, $21_3$ de positionnement, par leurs culs, de bouteilles ou canettes de bière B tenues en position au niveau de leur épaulement par un support à carcan 25 constitué par une réglette 26 à berceaux $27_1$, $27_2$, $27_3$ avec laquelle coopère une réglette de forme conjuguée 28 pouvant être fixée à la réglette 26 à l'aide d'écrous à oreille 29 vissés sur des tiges filetées 30 ancrées dans la réglette 26.

Sur l'aile 38 de petites dimensions du profilé 16, et dans l'alignement des coupelles 21, sont fixées des coupelles $35_1$, $35_2$, $35_3$, pour le positionnement de verres V lesquels reposent en outre dans leur partie haute, ou médiane, sur une réglette 36 analogue à la réglette associée au profilé 15.

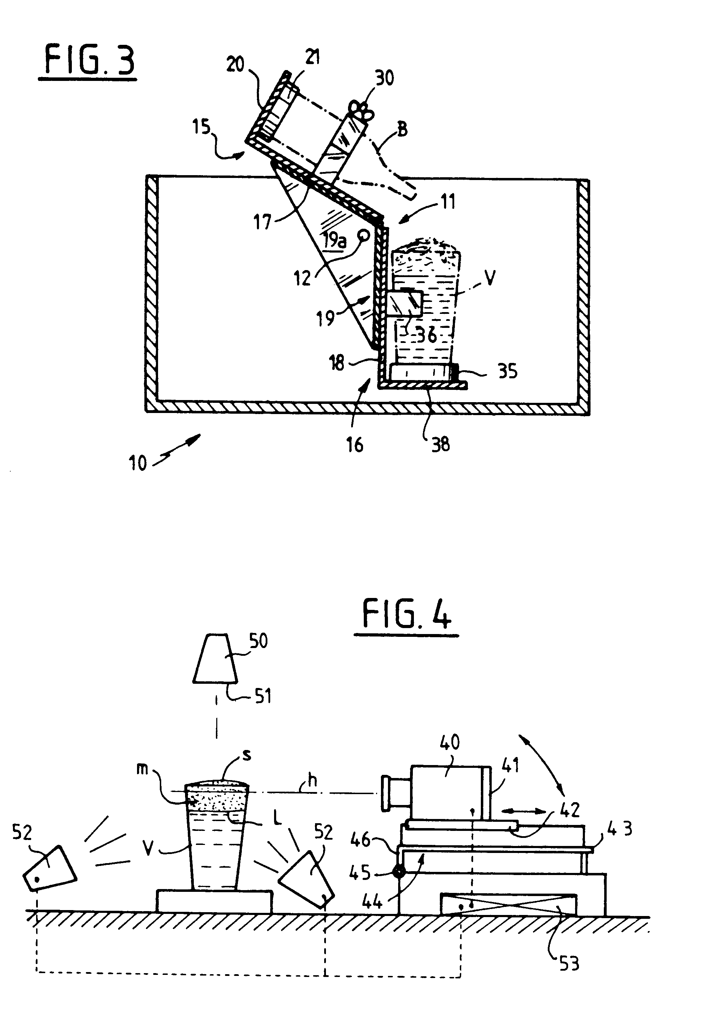

L'appareillage qui vient d'être décrit permet de transférer le contenu des bouteilles de bière B dans les verres V en commandant le moteur 13 pour faire pivoter l'équipage 11 de la position montrée sur la Figure 2 jusqu'à la position montrée sur la Figure 3, c'est-à-dire celle en laquelle l'aile 38 du profilé 16 est sensiblement parallèle au fond du bac 10. Par un choix approprié de la vitesse de rotation du moteur 13 et un écartement plus ou moins grand du débouché des bouteilles par rapport aux verres, - réglable à l'aide des boutonnières 22, 23 et des écrous à ailettes 37 - , on commande le transfert du contenu des bouteilles de bière dans les verres pour qu'il se forme au-dessus du niveau libre de la bière un chapeau de mousse, et cela de manière reproductible, aussi souvent que souhaité.

Si l'étude en fonction du temps de l'évolution des caractéristiques du chapeau de mousse doit être conduite non pas sur une ou des bières en bouteilles, mais sur de la bière en fût, l'invention prévoit d'associer à un robinet verseur usuel des moyens automatiques de commande pneumatique d'ouverture et de fermeture du robinet sous lequel est placé, à hauteur réglable, le verre destiné à recevoir la bière dont on souhaite étudier le chapeau de mousse, de sorte que le remplissage du verre, par soutirage du fût, est lui aussi réalisé de manière prédéterminée et reproductible.

Aussi bien dans le cas du dispositif de soutirage que dans celui de l'appareillage à bouteilles ou canettes, les essais sont conduits sur des verres ayant subi un traitement de lavage et de séchage particuliers, amenés à une température donnée et avec de la bière en fût ou en bouteille conservée pendant un temps et à une température prédéterminée.

On définit ainsi des conditions expérimentales de référence, reproductibles, à partir desquelles sont effectués des essais quantitatifs comme explicité ci-après en référence à la Figure 4 qui illustre schématiquement les moyens de mesure d'une installation selon l'invention.

Dans la forme de réalisation décrite et représentée, l'installation comprend un appareil de prise de vues 40, fixé sur une potence 41 elle-même montée à coulissement longitudinal sur une glissière 42 laquelle repose sur la tablette 43 d'une équerre 44, l'ensemble étant enfermé dans un boîtier, non représenté, à portes d'accès frontales et destiné à réduire l'effet d'éclairages parasites. L'équerre 44 est montée à pivotement autour d'un axe horizontal 45 à l'extrémité inférieure, sur le dessin, de l'aile 46 perpendiculaire à la tablette 43 de l'équerre 44. Cette organisation permet de réaliser des images du verre V dont le niveau de l'interface bière/mousse ou niveau libre de la bière est montré en L et dont le chapeau de mousse est montré en m, suivant un axe de prise de vue horizontal, montré par la ligne h ou suivant un axe sensiblement vertical lorsque l'on fait pivoter l'équerre 44 pour amener la tablette 43 dans un plan sensiblement vertical, après que l'appareil de prise de vues 40 ait été réglé en position par rapport au verre V à l'aide de la glissière 42 et de la potence 41.

Alors que le repérage dans le verre V du niveau libre L de la bière liquide peut être réalisé sans difficulté, il n'en est pas de même de la hauteur du sommet s du chapeau de mousse m dont l'affaissement au cours du temps est précisément une des variables que le procédé et l'installation selon l'invention permettent d'étudier.

C'est pourquoi l'invention propose de munir l'installation d'une source d'éclairement 50 à diaphragme 51 et de moyens de flash 52 dont le déclenchement est réglé, de même que celui de l'appareil de prise de vues 40, par un dispositif 53, le plus simplement un calculateur ou un microordinateur définissant une séquence d'intervalles de temps prédéterminés.

Dans une réalisation préférée, le diaphragme 51 ménage une fente rectiligne projetée sous forme d'un trait lumineux de forte intensité, lequel assure l'éclairage sans ambiguïté du sommet du chapeau de mousse dont la variation de hauteur, au cours du temps, peut être facilement repérée, nonobstant la présence sur la paroi interne de la partie haute du verre d'une "dentelle" de mousse au fur et à mesure que le chapeau diminue de hauteur.

Complémentairement, l'utilisation d'un diaphragme à fente rectiligne permet de déterminer la forme de la partie supérieure du chapeau de mousse, ce qui constitue un renseignement intéressant pour le spécialiste en brasserie.

Aux moyens formateurs d'images décrits ci-dessus sont associés, selon l'invention, des moyens de traitement permettant d'obtenir et, le cas échéant, traduire visuellement la variation des caractéristiques recherchées du chapeau de mousse.

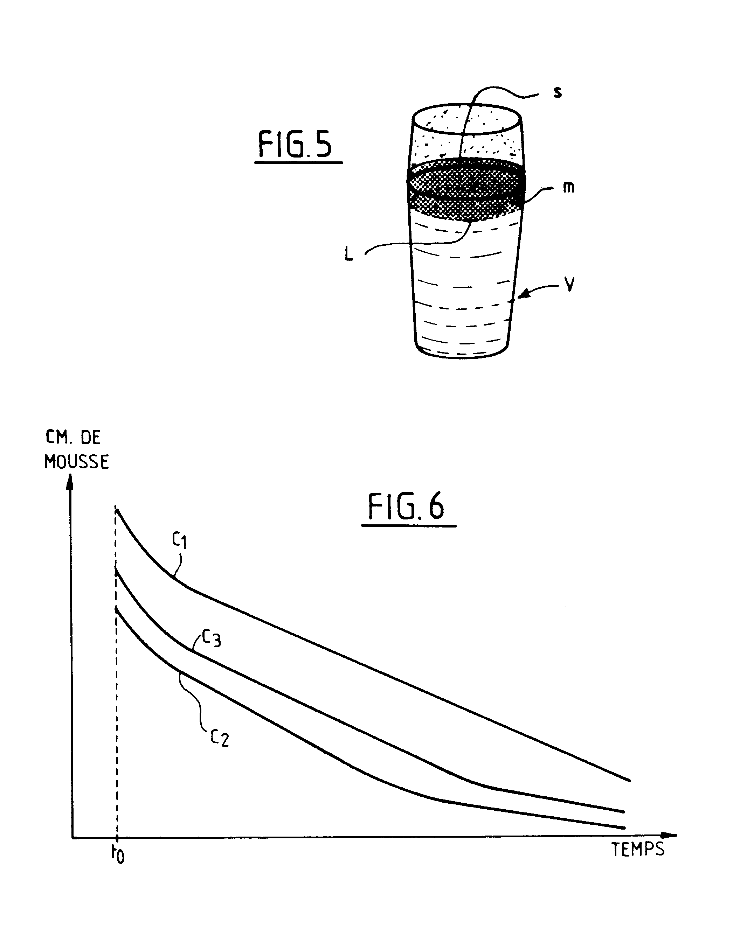

Dans une réalisation dans laquelle l'appareil de prise de vues 40 est un appareil photographique équipé d'un film à développement instantané, par exemple un film "Autoprocess 35" de la Société POLAROID, on obtient des diapositives dont l'analyse, sur un dispositif de visualisation de diapositives, permet de tracer des courbes comme celles montrées sur la Figure 6.

En variante, l'appareil de prise de vues est du type opto-électronique et les images sont analysées à l'aide d'un moyen du type télévision. Dans une telle réalisation, les résultats peuvent être transcrits automatiquement, à partir des informations fournies par un calculateur relié à l'aide d'un interface à la caméra et effectuant le traitement numérique de l'image vidéo, par exemple à l'aide d'un traceur de courbes ou d'une imprimante.

De façon plus précise l'invention prévoit, dans une telle réalisation, que la source d'éclairement 50 à diaphragme 51 projette un trait lumineux de forte intensité sur le sommet du chapeau de mousse $\overline{m}$ et que l'on substitue au moyen 52 de la réalisation décrite précédemment un moyen d'éclairage de la paroi du verre V constitué, par exemple, par une fibre optique dirigée de la partie inférieure du verre, en avant de celui-ci vers la zone L de l'interface bière/mousse. Comme dans la réalisation précédemment décrite, l'ensemble est enfermé dans un cabinet ou boîtier à parois internes noires qui enferme également l'appareil de prise de vues 40 réalisé, ici, en tant que caméra à semiconducteurs du type CCD (détecteur à charge couplée), reliée au microordinateur 53 par un interface approprié renfermant une mémoire suffisante pour stocker les 280 x 208 points constitutifs d'une image, laquelle peut être affichée sur un dispositif de visualisation et simultanément traitée par le microordinateur pour imprimer les valeurs numériques caractéristiques aux instants prédéterminés.

Quel que soit le moyen mis en oeuvre, l'invention permet de tracer, pour une bière donnée, la courbe représentative de la hauteur de mousse au-dessus du niveau libre de la bière, en fonction du temps écoulé à compter de l'opération de transvasement de la bière dans le verre à partir d'une bouteille, ou par soutirage hors d'un fût comme montré en $C_1$, $C_2$, $C_3$, Figure 6.

On constate alors que pour des bières conservées dans les mêmes conditions, en particulier de température, versées de manière identique dans des verres préalablement lavés, séchés et amenés à même température que celle de la bière, les hauteurs des chapeaux de mousse à l'instant initial, - comme montré par les ordonnées des points de même abscisse $t_o$ - diffèrent d'une bière lourde (Courbe $C_1$) à une bière légère (Courbe $C_2$), alors que toutes les courbes, y compris pour une bière de force moyenne (Courbe $C_3$) ont ensuite sensiblement la même allure, caractérisée en particulier par une partie médiane sensiblement linéaire.

La détermination précise de cette partie linéaire permet de quantifier la vitesse de décroissance du chapeau de mousse (pente de la Courbe représentative) et, ainsi, d'établir des critères de qualité objectifs tant au cours de la fabrication que de la dégustation ultérieure

Pour une réalisation à image optoélectronique, analysée à l'aide d'une caméra à semiconducteurs, le micro-ordinateur commande également une imprimante délivrant les Courbes C.

Dans une telle réalisation, le micro-ordinateur effectue également le traitement mathématique des courbes c'est-à-dire détermine les deux paramètres $H_O$ et K de la Loi mathématique de modélisation en fonction du temps $\underline{t}$ du type :

$$H = H_O e^{-Kt}$$

$H_O$ est la hauteur du chapeau de mousse à l'origine et K une constante associée au produit analysé.

**Revendications**

1. Procédé d'étude, en fonction du temps, de la variation de hauteur d'un chapeau de mousse de bière apparaissant à la partie supérieure d'un récipient transparent comme un verre ou analogue, après qu'y ait été versée la bière à partir d'une bouteille, canette, ou par soutirage d'un fût suivant lequel on détermine à l'aide de moyens optiques la hauteur de l'interface bière/mousse et celle du chapeau dans le récipient disposé verticalement, caractérisé en ce que l'on, projette sur le sommet du chapeau de mousse et selon un axe vertical un faisceau lumineux, en ce que l'on forme simultanément à des intervalles de temps prédéterminés et par des prises de vue effectuées transversalement au récipient des images du sommet dudit chapeau et du niveau de l'interface bière/mousse, et en ce que l'on traite lesdites images pour obtenir les variations des caractéristiques du chapeau de mousse, en particulier la vitesse de disparition dudit chapeau.

2. Procédé selon la revendication 1, caractérisé en ce que les images sont des images photographiques.

**3.** Procédé selon la revendication 1, caractérisé en ce que les images sont des images optoélectroniques, notamment des images de télévision.

**4.** Procédé selon l'une quelconque des revendications précédentes, caractérisé en ce que le faisceau lumineux est issu d'une fente rectiligne étroite, en ce que l'on éclaire aussi, avantageusement par un dispositif à fibre optique, la zone de l'interface bière/mousse et en ce que l'on traite les images obtenues à l'aide d'une caméra à semiconducteurs à laquelle est associé par l'intermédiaire d'un interface un microordinateur et, avantageusement, une imprimante à traceur de courbes.

**5.** Installation pour l'étude, en fonction du temps, des caractéristiques d'un chapeau de mousse apparaissant à la partie supérieure d'un récipient transparent comme un verre ou analogue après qu'y ait été versée la bière à partir d'une bouteille, canette, ou par soutirage d'un fût, avec des moyens optiques pour déterminer le niveau de l'interface bière/mousse et la hauteur de chapeau de mousse dans le récipient disposé verticalement, caractérisée en ce que lesdits moyens optiques comprennent, d'une part une source d'éclairement (50) à diaphragme (51) qui projette sur le sommet du chapeau de mousse et suivant un axe vertical un faisceau lumineux et, d'autre part, des moyens de formation d'images (40) propres à effectuer des prises de vue transversalement au récipient dudit niveau de l'interface bière/mousse et de la hauteur du sommet (s) du chapeau de mousse (m) à des instants pré-établis et des moyens de traitement desdites images associés aux moyens de formation d'images (40).

**6.** Installation selon la revendication 5, caractérisée en ce qu elle comprend en outre un appareillage pour remplir le verre (V) de bière de manière prédéterminée et reproductible, ledit appareillage comportant un équipage (11) en forme générale de lutrin à deux versants susceptible d'être animé d'un mouvement de pivotement autour d'un axe (12) parallèle à l'intersection desdits versants sous l'action d'un moteur (13) à vitesse réglable.

**7.** Installation selon la revendication 6, caractérisée en ce que l'équipage pivotant (11) comprend des moyens (21, 25, 35...) de positionnement relatif des bouteilles (B) et des verres (V) ainsi que des moyens de réglage (22, 23, 24, 37) de la distance relative des unes et des autres.

**8.** Installation selon l'une quelconque des revendications 5 à 7, caractérisée en ce que le diaphragme est un diaphragme linéaire à fente (51) étroite pour la projection d'un faisceau lumineux de forte intensité sur la partie supérieure du chapeau de mousse.

**9.** Installation selon la revendication 8, caractérisée en ce que les moyens formateurs d'images comprennent un appareil photographique (40) dont le déclenchement est synchrone de celui de moyens de flash (52), l'ensemble étant commandé suivant une séquence préprogrammée à l'aide d'un calculateur (53) ou analogue.

**10.** Installation selon l'une quelconque des revendications 5 à 7, caractérisée en ce que les moyens formateurs d'images sont des moyens opto-électroniques, avantageusement du type télévision comme une caméra à semiconducteurs auxquels est associé un micro-ordinateur pour la détermination des caractéristiques du chapeau de mousse (m) en fonction du temps, en particulier, la vitesse de diminution de la hauteur dudit chapeau.

**Claims**

**1.** A method of studying, as a function of time, the variation in height of a head of beer foam appearing in the upper part of a transparent receptacle such as a glass or the like, after the beer has been poured into the said receptacle from a bottle, can, or by being drawn from a vat, according to which optical means are employed to determine the height of the beer/foam interface and that of the head in the vertically disposed receptacle, characterised in that a beam of light is projected onto the top of the head of foam and along a vertical axis, and in that simultaneously at predetermined intervals of time and by means of photographs taken cross-wise through the receptacle, images of the said foam head and of the level of the beer/foam interface are formed, and in that the said images are processed in order to obtain the variations in the characteristics in the head of foam, particularly the rate at which the said head disappears.

**2.** A method according to Claim 1, characterised in that the images are photographic images.

**3.** A method according to Claim 1, characterised in that the images are optoelectronic images, in particular television images.

4. A method according to any one of the preceding Claims, characterised in that the light beam emanates from a narrow rectilinear slot and in that, advantageously by using a fibre optic apparatus, the zone of the beer/foam interface is illuminated and in that the images obtained by means of a semi-conductor camera to which a microcomputer and advantageously a graph tracing printer are associated via an interface are then processed.

5. An apparatus for studying, as a function of time, the characteristics of a head of foam appearing in the upper part of a transparent receptacle such as a glass or the like, after beer has been poured into it from a bottle or can, or by being drawn from a vat, with optical means of determining the level of the beer/foam interface and the height of the head of foam in the vertically disposed receptacle, characterised in that the said optical means comprise on the one hand a light source (50) with a diaphragm (51), which projects a beam of light onto the top of the foam head and along a vertical axis and, on the other, image-forming means (40) adapted to record images transversely to the receptacle to show the said level of the beer/foam interface and the height of the top (s) of the head of foam (m) at pre-established moments in time and means of processing the said images and associated with the image-forming means (40).

6. An apparatus according to Claim 5, characterised in that it further comprises an apparatus for filling the beer glass (V) in a predetermined and reproducible manner, the said apparatus comprising a generally lectern shaped arrangement (11) having two slopes and adapted to be driven with a pivoting motion about an axis (12) parallel with the intersection of the said slopes under the action of a regulable speed motor (13).

7. An apparatus according to Claim 6, characterised in that the pivoting arrangement (11) comprises means (21, 25, 35...) of relatively positioning bottles (B) and glasses (V) and means (22, 23, 24, 37) of regulating the relative distance between the former and the latter.

8. An apparatus according to any one of Claims 5 to 7, characterised in that the diaphragm is a diaphragm with a narrow linear slot (51) for projecting a high intensity beam of light onto the upper part of the head of foam.

9. An apparatus according to Claim 8, characterised in that the image-forming means comprise a photographic apparatus (40) the triggering of which is synchronised with that of flash means (52), the whole arrangement being controlled in accordance with a sequence pre-programmed by means of a computer (53) or the like.

10. An apparatus according to any one of Claims 5 to 7, characterised in that the image-forming means are optoelectronic means, advantageously of the television type, such as a semi-conductor camera with which a microcomputer is associated for determining the characteristics of the head of foam (M) as a function of the time and in particular the rate of diminution of the height of the said head.

**Patentansprüche**

1. Verfahren zur Überprüfung der zeitabhängigen Höhenveränderung einer Bierschaumhaube, die im oberen Teil eines transparenten Aufnahmebehälters, wie einem Glas oder ähnlichem entsteht, nachdem das Bier aus einer Flasche, Bierflasche oder durch Zapfen aus einem Faß eingeschenkt wurde, worauf mit optischen Mitteln die Höhe der Grenzfläche Bier/Schaum und jene der Haube an dem vertikal angeordneten Behälter festgestellt wird, **dadurch gekennzeichnet, daß** auf die Spitze der Schaumhaube und entlang einer vertikalen Achse ein Lichtstrahl gerichtet wird, daß in vorbestimmten Zeitintervallen gleichzeitig und durch seitlich von dem Behälter gemachte Aufnahmen Bilder der Spitze dieser Haube und des Niveaus der Grenzfläche Bier/Schaum hergestellt werden, und daß diese Bilder zum Feststellen der Veränderungen von Merkmalen der Schaumhaube, insbesondere die Geschwindigkeit beim Verschwinden dieser Haube verarbeitet werden.

2. Verfahren nach Anspruch 1, **dadurch gekennzeichnet, daß** die Bilder photographische Bilder sind.

3. Verfahren nach Anspruch 1, **dadurch gekennzeichnet, daß** die Bilder opto-elektronische Bilder, insbesondere Fernsehbilder sind.

4. Verfahren nach einem der vorigen Ansprüche, **dadurch gekennzeichnet, daß** der Lichtstrahl aus einem schmalen geraden Spalt ausgesandt wird, daß auch der Bereich der Grenzfläche Bier/Schaum, vorteilhafterweise mit einer faseroptischen Einrichtung beleuchtet und daß die erhaltenen Bilder mittels einer Halbleiter-Kammera verarbeitet werden, die über eine dazwischen liegende Schnittstelle mit einem Mikrocomputer und vorteilhafterweise einem Plotterschreiber verbunden ist.

5. Anlage zum Überprüfen der zeitabhängigen Merkmale einer Schaumhaube, die sich an der oberen Öffnung eines transparenten Behälters, wie einem Glas oder ähnlichem ausbildet, nachdem das Bier aus einer Flasche, Bierflasche oder durch Zapfen aus einem Faß eingeschenkt wurde, mit optischen Mitteln zum Bestimmen des Nieveaus der Grenzfläche Bier/Schaum und der Höhe der Schaumhaube in dem vertikal angeordneten Behälter, **dadurch gekennzeichnet, daß** die optischen Mittel einerseits eine Beleuchtungsquelle (50) mit einer Blende (51), die einen Lichtstrahl entlang einer vertikalen Achse auf die Spitze der Schaumhaube richtet, und andererseits Mittel zur Herstellung von Bildern (40), die dazu geeignet sind, zu voreingestellten Zeitpunkten seitliche Aufnahmen von dem Niveau der Grenzfläche Bier/Schaum und der Höhe der Spitze (s) der Schaumhaube (m) an dem Behälter herzustellen, und Mittel zum Verarbeiten der Bilder enthalten, die den Mitteln zur Herstellung der Bilder (40) zugeordnet sind.

6. Anlage nach Anspruch 5, **dadurch gekennzeichnet, daß** sie überdies eine Vorrichtung zum vorbestimmten und reproduzierbaren Füllen des Bierglases (V) aufweist, wobei diese Vorrichtung einen Teil (11) mit der allgemeinen Form eines Pultes mit zwei Neigungsflächen aufweist, der mittels eines Motors (13) mit regelbarer Geschwindigkeit um eine Achse (12) geschwenkt werden kann, die parallel zur Schnittlinie der Neigungsflächen verläuft.

7. Anlage nach Anspruch 6, **dadurch gekennzeichnet, daß** der schwenkbare Teil (11) Mittel (21, 25, 35, ...) zur relativen Positionierung von Flaschen (B) und Gläsern (V) und ebenso Mittel zum Einstellen (22, 23, 24, 37) des relativen Abstandes zueinander enthält.

8. Anlage nach einem der Ansprüche 5 bis 7, **dadurch gekennzeichnet, daß** die Blende eine lineare Blende in Form eines schmalen Spaltes (51) ist, um einen Lichtstrahl mit großer Intensität auf den oberen Teil der Schaumhaube zu richten.

9. Anlage nach Anspruch 8, **dadurch gekennzeichnet, daß** die Mittel zur Herstellung von Bildern einen Photoapparat (40) aufweisen, dessen Auslöser mit Mitteln zum Blitzen (52) synchronisiert ist, wobei die Anordnung gemäß einer vorprogrammierten Sequenz von einem Rechner (53) oder ähnlichem gesteuert wird.

10. Anlage nach einem der Ansprüche 5 bis 7, **dadurch gekennzeichnet, daß** die Mittel zur Herstellung von Bildern opto-elektronische Mittel, vorteilhafterweise wie beim Fernsehen eine Halbleiter-Kamera sind, denen ein Mikrocomputer zugeordnet ist, um die Merkmale der Schaumhaube (m) in Abhängigkeit von der Zeit, insbesondere die Geschwindigkeit der Verringerung der Höhe dieser Haube zu ermitteln.

FIG.1

FIG.2

FIG. 3

FIG. 4

FIG.5

CM. DE MOUSSE

FIG.6

$C_1$

$C_3$

$C_2$

$t_0$

TEMPS